# EUROPEAN PATENT SPECIFICATION

(11) **EP 1 945 157 B1**
(45) Date of publication and mention of the grant of the patent: **25.08.2010**
(21) Application number: 05772675.4
(22) Date of filing: 12.08.2005
(51) Int. Cl.: A61F 5/055, A61F 5/058

(54) **CORSET TO RELIEVE THE CERVICAL SPINE**
KORSETT ZUR ENTLASTUNG DER WIRBELSÄULE
APPAREIL ORTHOPÉDIQUE POUR SOULAGER LA COLONNE CERVICALE

(43) Date of publication of application: 23.07.2008
(73) Proprietor: TRANFIC, Vinko, 21000 Split (HR)
(72) Inventor: TRANFIC, Vinko, 21000 Split (HR)
(74) Representative: Pipan, Marjan
(86) International application number: PCT/HR2005/000043
(87) International publication number: WO 2007/020481

(56) References cited:
- DE-U1- 29 918 767
- US-A- 3 957 040
- US-A- 5 272 770
- US-A- 5 336 139
- US-B1- 6 368 295

## Description

### 1. FIELD OF APPLICATION

The invention relates to the corset that relieves the cervical spine. The corset comprises the head strap and the waist strap, mutually connected with strips that limit head bending and rotation.

### 2. TECHNICAL PROBLEM

The spine deceases are among the most common ones. Body movements and irregular postures in daily activities often overload the spine. Such movements and postures are to be avoided or limited to the minimum.

The deep muscles of the neck are important because they, by eccentric contractions, prevent the neck to bend forward and sideward and when the head is rotated. By their activity they particularly protect the neck lordosis, that is, forward curvature of the neck, thus protecting this fragile part of the spine. This way, musculature helps the disc and ligament soft tissues to absorb stress situations. Unfortunately, a large part of our daily activities include head movements and positions forward, sideward and rotation, where there is great danger of biomechanical damages of the discs.

Having this problem in mind, a corset is to be designed to enhance effects of the useful neck musculature and limit head movements forward, sideward and rotation.

### 3. STATE OF THE ART

Today there are numerous soft collars that fix the cervical spine. Also there are plastic collars that limit neck movements. In the state of the art there are known two particularly relevant patent documents:
US-A-5 336 and US-A-3 957 040, relating to limiting of head inclination and rotation. Although by their construction they are close to this invention, they do differ in essential details. This particularly relates to flexibility and complexity of embodiment stated in the referral documents.

### 4. DISCLOSURE OF THE INVENTION

The essence of the invention is the corset to relieve the cervical spine, comprising the head strap and the waist strap, mutually connected with one strip to limit the head movements forward, two strips to limit the movements sideward and two strips to limit the head rotation around the vertical axis. The strip length is adjustable, to fit the height of the person wearing it. By their construction, there are three corset embodiments: corset with strips with adjustable buckles, corset with strips with holes, and corset with strips with buckles and holes.

### 5. ILLUSTRATION DESCRIPTIONS

- Figure 1: shows the corset with strips with buckles, embodiment A - back perspective view.
- Figure 2: shows the corset with strips with holes, embodiment B - back perspective view.
- Figure 3: shows the corset with strips with buckles and holes, embodiment C - back perspective view.
- Figure 4: shows the corset shown in Fig. 1 on the user's body - back view.
- Figure 5: shows the corset with strips attached to a cap that acts as the head strap - side view.
- Figure 6: shows the corset with strips attached to a scarf that acts as the head strap - side view.
- Figure 7: shows the scarf corset on the user's body - back view.

### 6. DETAILED DESCRIPTION OF THE INVENTION EMBODIMENT

The corset to relieve the cervical spine, according to this invention, comprises: the head strap, the waist strap, the strips that limit head bending forward, the strips that limit head bending sideward, and the strips that limit head rotation around the vertical axis.

The head strap can be used directly or as part of a cap or head scarf.

By construction of the strips, there are three embodiments of the corset to relieve the cervical spine:
- corset A, with strips with buckles,
- corset B, with strips with holes, and
- corset C, with strips with buckles and holes.

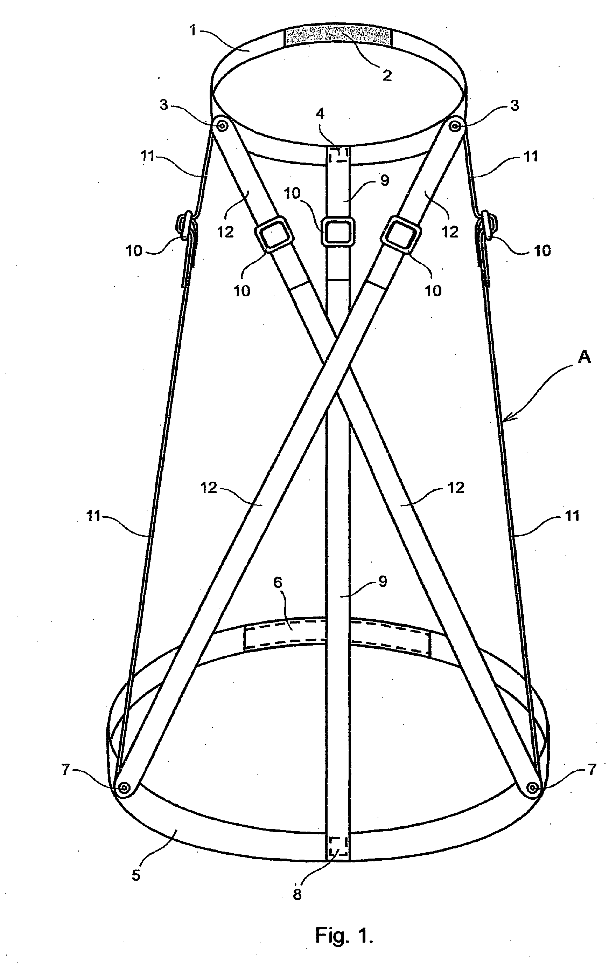

### Corset A, Figure 1, comprises:

- head strap 1, with fasteners 3, for detachable connecting the strips 11 and 12,
- waist strap 5, with fasteners 7, for detachable connecting the strips 11 and 12,
- stripe 9, with buckle 10 that is, in points 4 and 8, firmly fixed to straps 1 and 6,
- strips 11 and 12, with buckles 10, movably fixed to fasteners 3 and 7,
- strip 12, with buckles 10, crossed and detachably fixed to fasteners 3 and 7, over the back.

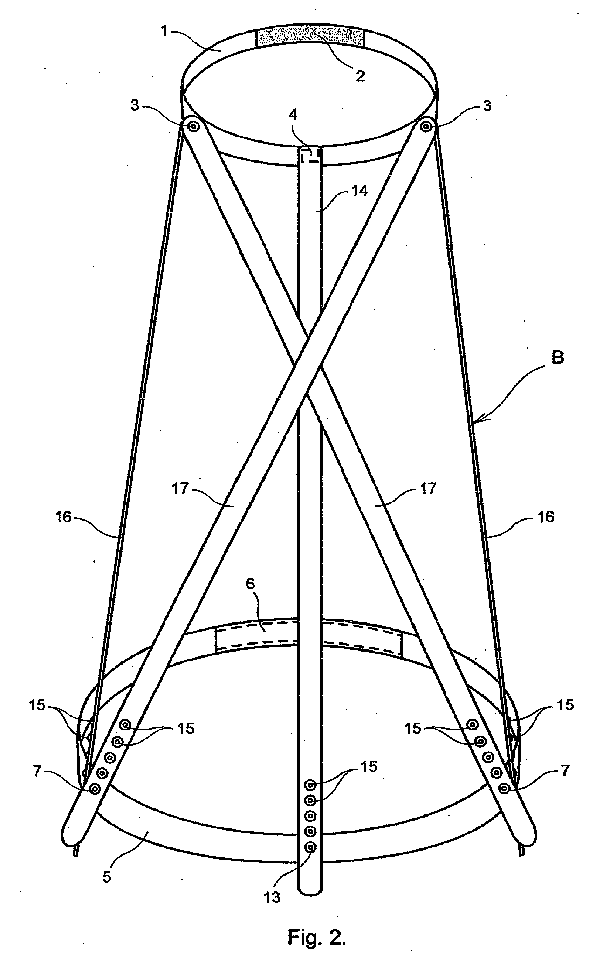

### Corset B, Figure 2, comprises:

- head strap 1, with fasteners 3, for detachable connecting the strips 16 and 17,
- waist strap 5, with fasteners 7 and 13, for detachable connecting the strips 16 and 17,
- strip 14, with holes 15 for detachable connecting the strap 13,
- strips 16 and 17, with holes 15, for detachable connecting the strip 7.

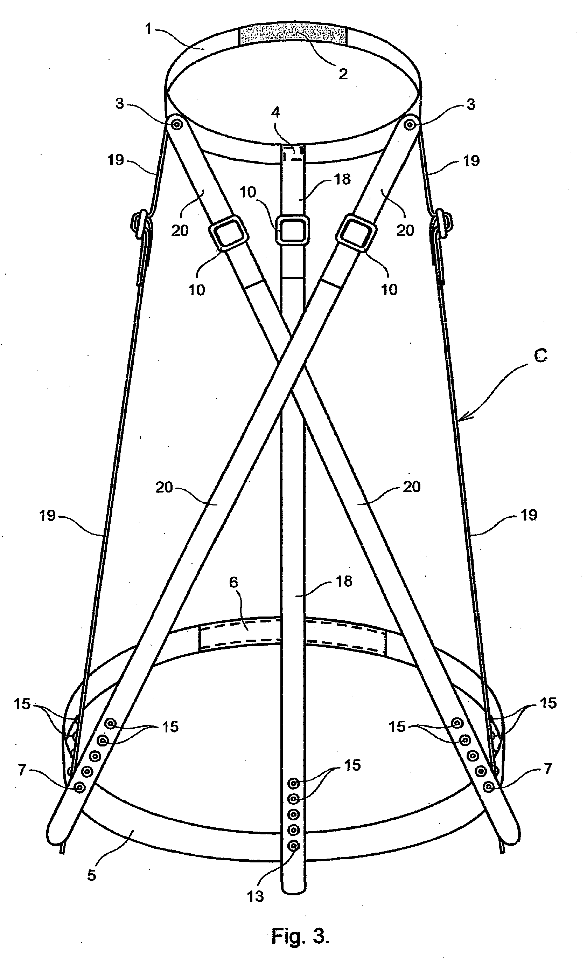

### Corset C, Figure 3, comprises:

- head strap 1, with fasteners 3, for detachable connecting the strips 19 and 20,
- waist strap 5, with fasteners 7 and 13, for detachable connecting the strips 18, 19 and 20,
- strips 18, 19 and 20, with buckles 10 and holes 15, for connecting to the strips 7 and 13,
- strips 20, crossed over the back.

The strap 1 is fitted to the user's head, and fixed with Velcro strap 2, whereas the strap 5 is fitted around the waist and fixed with Velcro strap 6.

The strips 9, 11 and 12 in the A embodiment, arid strips 18, 19 and 20 in the C embodiment are made of two parts, mutually connected with the buckle 10 that enables their length adjustment. The holes 15 are made in the lower part of the strips, enabling adjusting the strips to the user's needs.

The strips 9, 14 and 18 are mounted across the back, to limit the head bending forward. The strips 11, 16 and 19 are to limit the head bending to the right and the left sides. The strips 12, 17 and 20 are crossed and to limit rotation of the head around the vertical axis.

The fasteners 3 are fixed to the head strap 1 at its opposite sides.

The fasteners 7 are fixed to the waist strap 5 at its opposite sides.

The fastener 13 is fixed to the middle of the back part of the waist strap.

The fasteners 3, 7 and 13, with one or more openings 15, and buckles 10, may be embodied in any of the already known ways.

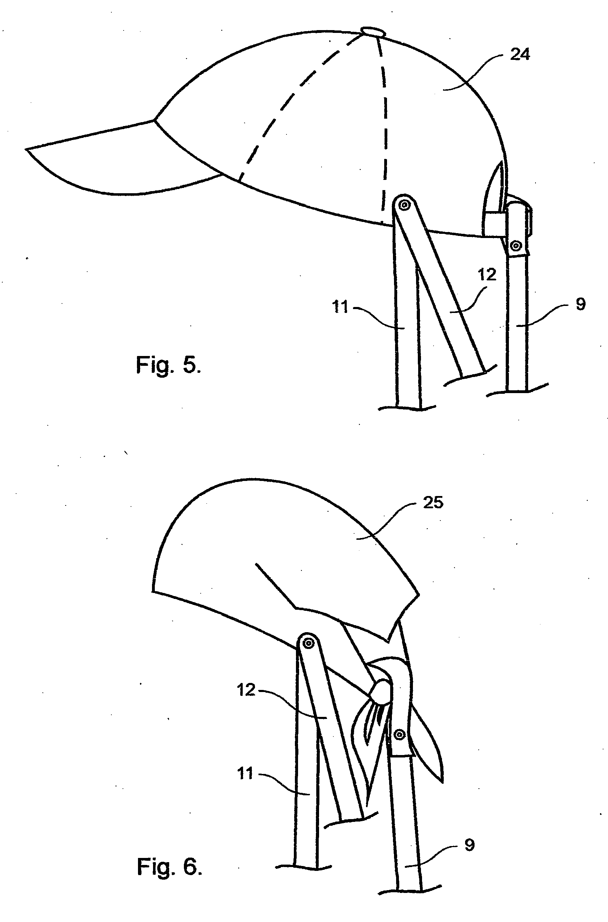

### Cap functioning as the head strap, Figure 5

Figure 5 shows fitting of the strips, to limit the head bending and rotation, to a cap. The cap may be of any shape, however, its function, with fitted strips, will remain unchanged.

### Head scarf functioning as the head strap, Figure 6

Figure 6 shows fitting of the strips, to limit the head bending and rotation, to a head scarf. The scarf may be of any shape, however, its function, with fitted strips, will remain unchanged.

The strips may be fixed to a cap or scarf in any of the already known ways.

### Functioning of the corset

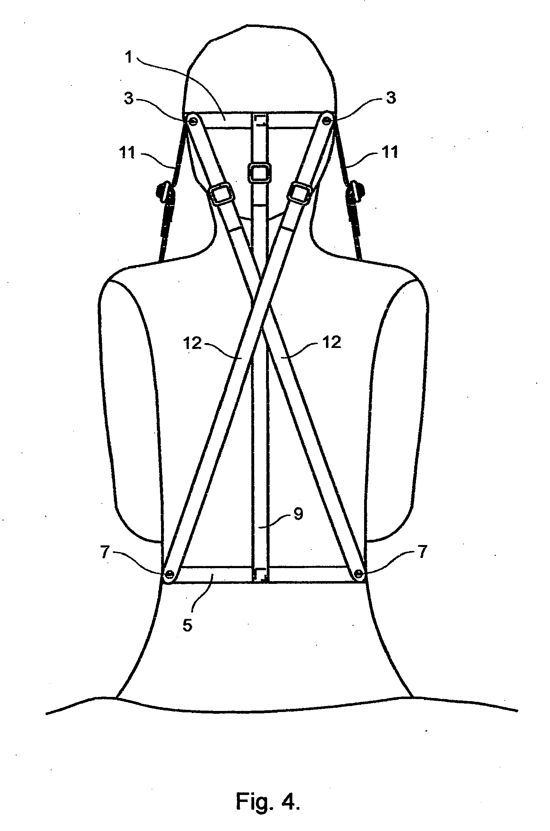

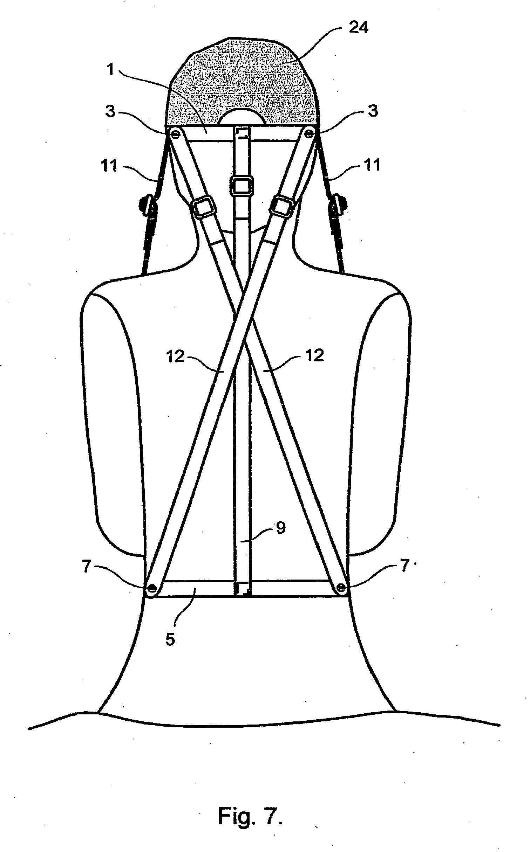

The corset that relieves the cervical spine is fitted around the head and around the waist, as shown in Figures 4 and 7, depending on whether the head strap is applied directly to the head or a cap or head scarf embodiment is used. The head strap is fixed over the forehead, and the waist strap around the body, in the lumbar region. After this, and depending on the discomfort, adequate strip or strips are fitted and adjusted.

The corset that relieves the cervical spine functions when its user, by his or her movements, brings the cervical spine into an unfavourable position. This results in a significant fixation that enhances effects of eccentric contraction of the deep neck muscles. This, in head bending and rotation, significantly diminishes the aggravating effects to the cervical spine.

In such situations, the strips stretch and prevent overloading of the neck and damages to the discs. Simultaneously, the eccentric contraction of the neck muscles is supported, which decreases fatigue which, in turn, enables longer performing of jobs that cause the neck to bend. Furthermore, in damaged cervical spines, adjusting of the strips enables blocking that very movement that aggravates the situation.

### 7. INVENTION APPLICATION

The corset that relieves the cervical spine can be used:
- in physical medicine,
- for educational purposes - learning of proper movements,
- prevention in sports (rowing, sailing, tennis, basketball, ...), and
- in performing daily and other actions that require bending of the cervical spine forward, since it releases the tender, soft tissues of the neck and protects them from injuries.

## Claims

1. Corset that relieves the cervical spine, consists of a head strap (1) and a waist strap (6), whereby the head strap and the waist strap are connected with a first strip (9,14,18), in use passing over the back of a user and limiting bending of the head forward **characterized in that** said head and waist strap are further connected at lateral sides with a set of second strips (11,16,19) limiting bending of the head sideward, ***wherein**,* with regard to the construction and ways of connecting said first and second strips to the head strap and the waist strap there are three corset embodiments: corset (A) with buckles (10) on the first and second strips, corset (B) with holes (15) on the first and second strips, and corset (C) with buckles (10) and holes (15) on the first and second strips; all three corset embodiments suitable to relieve the cervical spine (A, B and C) comprise third strips (12, 17 and 20) limiting rotation of the head around its vertical axis and they are crossed over the user's back; in case of the corset (A) the first strip (9)
is fixed firmly with one end in a first position (4) to the head strap (1) and with the other end is fixed firmly in a second position (8) to the waist strap (5), the second strips (11)
and third strips (12) are fixed movably with one end via fasteners (3) to the head strap and with the other end via the fasteners (7) fixed movably to the waist strap; in case of the corset (B) the first strip (14) is with one end firmly fixed to the head strap and with the other end, by holes (15) and via the fastener (13) fixed movably to the waist strap, the second strips (16) and third strips (17)
are with one end fixed movably via the fasteners (3) to the head strap, and with the other end through the holes (15) and via the fasteners (7) are movably fixed to the waist strap; in case of the corset (C) the first strip (18) is with one end fixed firmly to the head strap and with the other end with the holes (15) and via the fastener (13) fixed movably to the waist strap, and the second strips (19) and third strips (20)
are with one end via the fastener (3) third fixed movably to the head strap and with the other end with the holes (15) and via the fastener (7) fixed movably to the waist strap.

2. Corsets, as claimed in Claim 1, ***wherein*** the head strap (1) has a Velcro strap (2) for adjusting and tightening said head strap around the user's head.

3. Corsets, as claimed in Claim 1, ***wherein*** the waist strap (5) has a Velcro strap (6) for adjusting and tightening said waist strap around the user's waist.

4. Corsets, as claimed in Claim 1, ***wherein*** the said fasteners (3) are fixed to the outer surface of the head strap (1), at opposite sides.

5. Corsets, as claimed in Claim 1, ***wherein*** the said fastener (13) is fixed in the middle of a back part of the waist strap (5).

6. Corsets, as claimed in Claim 1, ***wherein*** the said fasteners (7) are fixed to the waist strap (5), at opposite sides.

7. Corsets, as claimed in Claim 1, ***wherein*** the first strips (14 and 18)
the second strips (16 and 19)
and the third strips (17 and 20) each have in a lower part which has at least one hole (15) that enable adjustment of strip length when fitted to the fasteners (7 and 13) on the waist strap.

## Patentansprüche

1. Korsett zur Entlastung der Wirbelsäule, das einen Riemen für den Kopf (1) und einen für die Taille (6) hat, wobei der Riemen für den Kopf und der Riemen für die Taille durch das erste Band (9, 14,18) verbunden sind, das bei Gebrauch über den Rücken des Benutzers läuft und die Neigung des Kopfes nach vorne verhindert, **dadurch** charakterisiert, dass der besagte Riemen für den Kopf und der Riemen für die Taille des Weiteren an den Seiten durch eine Gruppe weiterer Bänder (11, 16, 19) verbunden sind, die die seitliche Neigung des Kopfes verhindern, **dadurch gekennzeichnet, dass** wir im Hinblick auf die Konstruktion und die Art der Verbindung der besagten ersten und zweiten Bänder am Riemen für den Kopf und am Riemen für die Taille drei Ausführungen des Korsetts unterscheiden, nämlich: Korsett (A) mit Ringen (10) an den ersten und zweiten Bändern, Korsett (B) mit Öffnungen (15) an den ersten und zweiten Bändern und Korsett (C) mit Ringen (10) und Öffnungen (15) an den ersten und zweiten Bändern; dass alle drei Ausführungen des Korsetts zur Entlastung der Wirbelsäule (A, B und C) dritte Bänder (12, 17 und 20) für die Begrenzung der Drehung des Kopfes um die senkrechte Achse haben, die im Rücken des Benutzers gekreuzt sind; dass beim Korsett (A) das erste Band (9) mit einem Ende fest in der ersten Position (4) am Riemen für den Kopf (1) und mit dem anderen Ende fest in der zweiten Position (8) an den Riemen für die Taille (5) festgebunden ist, dass die zweiten Bänder (11) und die dritten Bänder (12) beweglich über Schnallen (3) mit einem Ende am Riemen für den Kopf und mit dem anderen Ende über Schnallen (7) beweglich am Riemen für die Taille festgebunden ist; dass beim Korsett (B) das erste Band (14) mit einem Ende fest an den Riemen für den Kopf, und mit dem anderen Ende durch die Öffnungen (15) über eine Schnalle (13) beweglich an den Riemen für die Taille festgebunden ist, dass die zweiten Bänder (16) und dritten Bänder (17) mit einem Ende über Schnallen (3) beweglich an den Riemen für den Kopf und mit dem anderen Ende durch die Öffnungen (15) über Schnallen (7) beweglich an den Riemen für die Taille festgebunden ist; dass beim Korsett (C) das erste Band (18) mit einem Ende fest an den Riemen für den Kopf und mit dem anderen durch die Öffnungen (15) über eine Schnalle (13) beweglich an den Riemen für die Taille festgebunden ist, dass die zweiten Bänder (19) und die dritten Bänder (20) mit einem Ende über Schnallen (3) beweglich an den Riemen für den Kopf und mit dem anderen Ende durch die Öffnungen (15) über Schnallen (7) beweglich an den Riemen für die Taille festgebunden sind.

2. Korsette, nach Anspruch 1, **dadurch gekennzeichnet, dass** der Riemen für den Kopf (1) einen eingebauten Klettverschluss (2) für die Einstellung des besagten Riemens für den Kopf für den Umfang des Kopfs des Benutzers hat.

3. Korsette, nach Anspruch 1, **dadurch gekennzeichnet, dass** der Riemen für die Taille (5) einen eingebauten Klettverschluss (6) für die Einstellung des besagten Riemens für die Taille für den Umfang der Taille des Benutzers hat.

4. Korsette, nach Anspruch 1, **dadurch gekennzeichnet, dass** die genannten Schnallen (3) an der Außenfläche des Riemens für den Kopf (1) an den diametral entgegen gesetzten Seiten befestigt sind.

5. Korsette, nach Anspruch 1, **dadurch gekennzeichnet, dass** die genannte Schnalle (13) an der Mitte des hinteren Teils des Riemens für die Taille (5) befestigt ist.

6. Korsette, nach Anspruch 1, **dadurch gekennzeichnet, dass** die genannten Schnallen (7) am Riemen für die Taille (5) an den diametral entgegen gesetzten Seiten befestigt sind.

7. Korsette, nach Anspruch 1, **dadurch gekennzeichnet, dass** die ersten Bänder (14 und 18), die zweiten Bänder (16 und 19) und die dritten Bänder (17 und 20) alle im unteren Teil mindestens eine Öffnung (15) haben, die die Einstellung der Bandlänge ermöglichen, wenn sie an den Schnallen (7 und 13) am Riemen für die Taille angebracht werden.

## Revendications

1. Appareil orthopédique pour soulager la colonne cervicale, constitué d'une ceinture pour la tête (1) et d'une ceinture pour la taille (6), la ceinture pour la tête et la ceinture pour la taille étant reliées avec la première bande (9, 14, 18), laquelle lors de l'utilisation passe à travers le dos de l'utilisateur et limite l'inclination de la tête en avant, **caractérisé par le fait que** la ceinture pour la tête et la ceinture pour la taille précitées sont ultérieurement reliées aux côtés latérales avec une série d'autres bandes (11, 16, 19) limitant l'inclination de la tête latéralement, **caractérisé par la fait que**, compte tenu de la construction et le mode de connexion des premières et des deuxièmes bandes précitées sur la ceinture pour la tête et la ceinture pour la taille, on distingue trois réalisations de l'appareil, soit: l'appareil orthopédique pour soulager la colonne cervicale (A) avec les bagues (10) sur les premières et les deuxièmes bandes, l'appareil orthopédique pour soulager la colonne cervicale (B) avec les ouvertures (15) sur les premières et les deuxièmes bandes et l'appareil orthopédique pour soulager la colonne cervicale (C) avec les bagues (10) et les ouvertures (15) sur les premières et les deuxièmes bandes; que toutes les trois réalisations de l'appareil orthopédique pour soulager la colonne cervicale (A, B i C) ont les troisièmes bandes (12, 17 i 20) pour limiter la rotation de la tête sur l'axe vertical, qui sont croisées à travers le dos de l'utilisateur; que chez l'appareil orthopédique pour soulager la colonne cervicale (A) la première bande (9) est par une de ses extrémités fermement connectée dans la première position (4) avec la ceinture pour la tête (1), et par l'autre fermement connectée dans la deuxième position (8) avec la ceinture pour la taille (5), que les deuxièmes bandes (11) et les troisièmes bandes (12) sont connectées de manière réglable au moyen des boucles (3) par l'une des extrémités sur la ceinture pour la tête, et par l'autre extrémité, par les boucles (7) sont connectées de manière réglable sur la ceinture pour la taille; que chez l'appareil orthopédique pour soulager la colonne cervicale (B) la première bande (14) est fermement connectée avec la ceinture pour la tête, et par son autre extrémité, par les ouvertures (15), à travers le boucle (13), est connectée de manière réglable avec la ceinture pour la taille, que les deuxièmes bandes (16) et les troisièmes bandes (17) par l'une de leurs extrémités, à travers les boucles (3), sont connectées de manière réglable avec la ceinture pour la tête, et par l'autre extrémité, par les ouvertures (15), à travers les boucles (7), aussi de manière réglable avec la ceinture pour la taille; que chez l'appareil orthopédique pour soulager la colonne cervicale (C) la première bande (18) est connectée avec l'une de ses extrémités avec la ceinture pour la tête et par son autre extrémité, par les ouvertures (15), à travers le boucle (13), est connectée de manière réglable avec la ceinture pour la taille, que les deuxièmes bandes (19) et les troisièmes bandes (20) sont par l'une des leurs extrémités, à travers le boucle (3) connectées de manière réglable avec la ceinture pour la tête, et par l'autre extrémité, par les ouvertures (15), à travers le boucle (7), sont connectées de manière réglable avec la ceinture pour la taille.

2. Les appareils orthopédiques pour soulager la colonne cervicale, selon la revendication 1, **sont caractérisés par le fait que**, sur la ceinture pour la tête (1) se trouve une attache velcro (2), pour le réglage de ladite ceinture pour la tête selon le volume de la tête de l'utilisateur.

3. Les appareils orthopédiques pour soulager la colonne cervicale, selon la revendication 1, **sont caractérisés par le fait que**, sur la ceinture pour la taille (5) se trouve une attache velcro (6), pour le réglage de ladite ceinture pour la taille selon le volume de la teille de l'utilisateur.

4. Les appareils orthopédiques pour soulager la colonne cervicale, selon la revendication 1, **sont caractérisés par le fait que**, que lesdites boucles (3) sont fixées sur la surface extérieure de la ceinture pour la tête (1), en points diamétralement opposés.

5. Les appareils orthopédiques pour soulager la colonne cervicale, selon la revendication 1, **sont caractérisés par la fait que**, que ladite boucle (13) est fixée au milieu de la partie postérieure da la ceinture pour la taille (5).

6. Les appareils orthopédiques pour soulager la colonne cervicale, selon la revendication 1, **sont caractérisés par le fait que**, que lesdites boucles (7) sont fixées sur la ceinture pour la taille (5), en points diamétralement opposés.

7. Les appareils orthopédiques pour soulager la colonne cervicale, selon la revendication 1, **sont caractérisés par le fait que**, que les premières bandes (14 et 18), deuxièmes bandes (16 et 19) et les troisièmes bandes (17 et 20), toutes ont dans leur partie inférieure au moins une ouverture (15) rendant possible le réglage de la longueur des bandes quand elles sont posées dans les boucles (7 et 13) sur la bande pour la taille.
